(19) Europäisches Patentamt
European Patent Office
Office européen des brevets

(11) EP 4 129 408 A1

(12) EUROPEAN PATENT APPLICATION
published in accordance with Art. 153(4) EPC

(43) Date of publication:
**08.02.2023 Bulletin 2023/06**

(21) Application number: **21774006.7**

(22) Date of filing: **22.03.2021**

(51) International Patent Classification (IPC):
***A61P 43/00** (2000.01)* ***A61P 17/00** (2000.01)*
***A23L 33/105** (2016.01)* ***A23L 33/125** (2016.01)*
***A61K 31/7048** (2000.01)*

(52) Cooperative Patent Classification (CPC):
**A23L 33/105; A23L 33/125; A61K 31/7048; A61P 17/00; A61P 43/00**

(86) International application number:
**PCT/JP2021/011634**

(87) International publication number:
**WO 2021/193498 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.03.2020 JP 2020054505**

(71) Applicant: **Toyo Sugar Refining Co., Ltd.**
**Tokyo 103-0016 (JP)**

(72) Inventors:
- **NAKANISHI Akihito**
  **Ichihara-shi, Chiba 290-0046 (JP)**
- **TANDIA Mahamadou**
  **Noda-shi, Chiba 278-0027 (JP)**

(74) Representative: **Dehns**
**St. Bride's House**
**10 Salisbury Square**
**London EC4Y 8JD (GB)**

# (54) COLLAGENASE ACTIVITY INHIBITOR

(57) An objective of the present invention is to provide a collagenase activity inhibitor exhibiting an excellent collagenase activity inhibition action. A collagenase activity inhibitor, containing at least one component (A) selected from α-glucosylrutin, α-glucosyl hesperidin and α-glucosyl naringin.



Processed by Luminess, 75001 PARIS (FR)

## Description

### Technical Field

The present invention relates to a collagenase activity inhibitor.

### Background Art

It has been clarified that matrix metalloproteinases (MMPs) decompose extracellular matrices and are also involved in a variety of physiological phenomena such as the activation of physiologically active peptides such as cytokine and related to a variety of disease states. Among MMPs, collagenases have been reported to be involved in a variety of diseases such as articular rheumatism, osteoarthritis, osteoporosis, metastasis of cancer cells, development of ulcer, periodontitis and cavity formation.

Collagen in the skin is important in maintaining skin elasticity or tension. Acceleration of collagenase expression and a resultant decrease in the amount of collagen in the skin due to the influence of, for example, ultraviolet rays are considered as one of the causes for wrinkles or sagging of the skin. Therefore, inhibition of the activity of collagenase is important in the prevention of wrinkles or sagging, that is, the prevention of skin aging, and thus a variety of active components have been thus far reported.

For example, a collagenase activity inhibitor containing one or more botanical extracts selected from raspberry, clove, loquat, Morus alba and Chinese milk vetch (Patent Literature 1), a collagenase activity inhibitor containing a botanical extract such as grape leaf or Zanthoxylum piperitum (Patent Literature 2), a collagenase activity inhibitor with which a Punica granatum flower extract is blended (Patent Literature 3), a collagenase activity inhibitor containing a Duabanga extract (Patent Literature 4), a collagenase activity inhibitor containing a tea flower extract (Patent Literature 5), and a collagenase activity inhibitor containing a Pseudocydonia sinensis extract as an active component (Patent Literature 6) are known. However, all of the collagenase activity inhibitors have a weak aging prevention action and are not always satisfactory.

### Citation List

### Patent Literature

Patent Literature 1
JP2003-183122A
Patent Literature 2
JP2003-342123A
Patent Literature 3
JP2006-225286A
Patent Literature 4
JP2008-74728A
Patent Literature 5
JP2011-11991A
Patent Literature 6
JP2016-104707A


### Summary of Invention

### Technical Problem

An objective of the present invention is to provide a collagenase activity inhibitor exhibiting an excellent collagenase activity inhibition action.

### Solution to Problem

The present inventors carried out intensive studies to solve the above-described problem. As a result, the present inventors have found that the above-described problem can be solved by a collagenase activity inhibitor having the following configuration and completed the present invention. The present invention provides, for example, the following

[1] to [5].

[1] A collagenase activity inhibitor, containing at least one component (A) selected from α-glucosylrutin, α-glucosyl hesperidin and α-glucosyl naringin.
[2] The collagenase activity inhibitor according to [1], in which the component (A) contains at least one selected from α-monoglucosylrutin, α-monoglucosyl hesperidin and α-monoglucosyl naringin.
[3] The collagenase activity inhibitor according to [1], in which a content of the component (A) is 70 mass% or more.
[4] The collagenase activity inhibitor according to [2], in which a content of the component (A) is 70 mass% or more.
[5] The collagenase activity inhibitor according to [1], in which the collagenase activity inhibitor inhibits an activity for decomposition of Type I collagen.

Advantageous Effect of Invention

According to the present invention, it is possible to provide a collagenase activity inhibitor exhibiting an excellent collagenase activity inhibition action.

Brief Description of Drawings

[Figure 1] Figure 1 is a graph showing the collagenase activity inhibition rates of α-glucosylrutin (αG rutin), α-glucosyl hesperidin (αG hesperidin) and α-glucosyl naringin (αG naringin).
[Figure 2] Figure 2 is a graph showing the collagenase activity inhibition rates of ascorbic acid, α-glucosylrutin (αG rutin), α-glucosyl hesperidin (αG hesperidin) and α-glucosyl naringin (αG naringin).
[Figure 3] Figure 3 is a graph showing the collagenase activity inhibition rates of sodium ascorbate, α-glucosylrutin (αG rutin), α-glucosyl hesperidin (αG hesperidin) and α-glucosyl naringin (αG naringin).

Description of Embodiment

Next, a collagenase activity inhibitor of the present invention will be specifically described.

<Component (A)>

A collagenase activity inhibitor of the present invention contains at least one component (A) selected from α-glucosylrutin, α-glucosyl hesperidin and α-glucosyl naringin. The component (A) may contain any one or arbitrarily selected two of α-glucosylrutin, α-glucosyl hesperidin and α-glucosyl naringin or may contain all three of α-glucosylrutin, α-glucosyl hesperidin and α-glucosyl naringin. In particular, the collagenase activity inhibitor preferably contains α-glucosyl hesperidin or α-glucosyl naringin as the component (A) since this has a strong collagenase activity inhibition effect even at a relatively low concentration.
The component (A) preferably contains at least one selected from α-monoglucosylrutin, α-monoglucosyl hesperidin and α-monoglucosyl naringin from the viewpoint of the collagenase activity inhibition effect.
The content of the component (A) in the collagenase activity inhibitor is not particularly limited. For example, the lower limit of the content of the component (A) in the collagenase activity inhibitor of the present invention is, for example, 30 mass%, 40 mass%, 45 mass%, 50 mass%, 60 mass%, 70 mass% or 80 mass%. In addition, the upper limit of the content of the component (A) in the collagenase activity inhibitor of the present invention is, for example, 100 mass%, 99 mass%, 98 mass%, 95 mass%, 90 mass% or 85 mass%. As the range of the content of the component (A) in the collagenase activity inhibitor of the present invention, a range can be arbitrarily set by arbitrarily combining the above-described lower limit and upper limit, and, for example, a range of 30 to 100 mass%, 60 to 100 mass%, or 60 to 90 mass% can be set. From the viewpoint of the collagenase activity inhibition effect, the content of the component (A) in the collagenase activity inhibitor of the present invention is preferably 70 mass% or more.

<α-Glucosylrutin>

α-Glucosylrutin (also referred to as α glucosylrutin) is a collective term of compounds in which one or more molecules of glucose are added to a glucose residue in a rutinose residue in rutin by an α-$1_{\rightarrow}4$ linkage. α-Glucosylrutin in the present invention may be composed only of one compound having such a structure or may be a mixture of two or more compounds having such a structure.
α-Glucosylrutin can be represented by the following formula (1). In the formula (1), n is 0 or an integer of 1 or

more, for example, an integer of 1 to 19.

[Chem. 1]

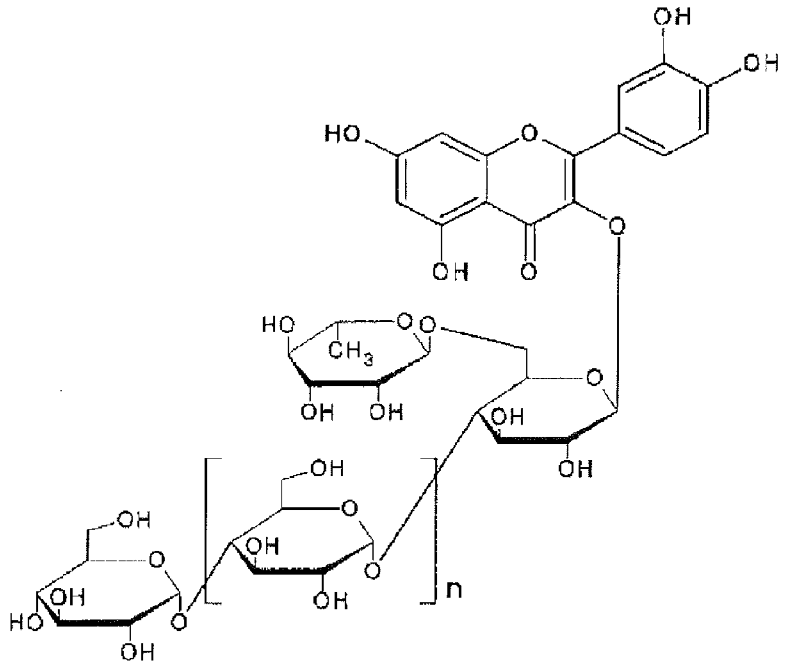

・・・ (1)

α-Glucosylrutin is a compound that is contained as a main component in a product known as "enzyme-treated rutin" (also called "transglycosylated rutin" in some cases). α-Glucosylrutin to which only one molecule of glucose is linked is called "a-monoglucosylrutin", and α-glucosylrutin to which two or more molecules of glucose are linked is called "α-polyglucosylrutin". That is, in the formula (1), α-monoglucosylrutin is a compound for which n is 0, and α-polyglucosylrutin is ordinarily a compound for which n is 1 to 19.

The enzyme-treated rutin is an aggregate of compounds formed by enzyme treatment of sugar of rutin and normally contains a mixture of compounds in which the number of glucose molecules linked to rutin is different from one another, for example, a mixture composed of α-monoglucosylrutin and α-polyglucosylrutin. In addition, the enzyme-treated rutin is ordinarily produced by enzyme treatment and thus contains unreacted rutin or a different derivative, for example, isoquercitrin in some cases. Isoquercitrin is a compound in which β-D-glucose is linked to a hydroxyl group at the position 3 of a quercetin skeleton, in other words, a compound in which a rhamnose residue in a rutinose residue of rutin is cut.

The enzyme-treated rutin is, for example, a product that is obtained by causing a glycosyltransferase (an enzyme having a function of adding glucose to rutin such as cyclodextrin glucanotransferase (CGTase, EC2.4.1.19)) to act on rutin in the coexistence of an α-glucosyl sugar compound (such as cyclodextrin or partially degraded starch) (called "first enzyme-treated rutin" in the present specification).

The first enzyme-treated rutin is a composition containing a variety of α-glucosylrutin in which the number of linked glucose molecules is different from one another, that is, an aggregate composed of α-monoglucosylrutin and α-polyglucosylrutin, and rutin that is an unreacted substance. If necessary, the first enzyme-treated rutin is purified using, for example, a porous synthetic adsorbent and an appropriate eluent, whereby a first enzyme-treated rutin in which the glycosyl donor and other impurities are removed, furthermore, the content of rutin is decreased and the purity of α-glucosylrutin is increased (α-glucosylrutin purified substance) can be obtained.

In addition, when the first enzyme-treated rutin is treated with an enzyme having a glucoamylase activity that cuts an α-1,4-glucoside linkage by glucose unit, for example, glucoamylase (EC3.2.1.3), and, in α-glucosylrutin to which a plurality of glucose molecules has been added, glucose residues directly added to glucose residues (in a rutinose residue) of rutin itself are cut except only one glucose residue, enzyme-treated rutin containing a large amount of α-monoglucosylrutin (called "second enzyme-treated rutin" in the present specification) can be obtained. There is no case

where the glucose residues in the rutinose residue directly linked to a quercetin skeleton are cut from the quercetin skeleton due to this enzyme treatment.

In the collagenase activity inhibitor of the present invention, when, for example, the effect of the present invention is taken into account, enzyme-treated rutin that is a composition containing α-glucosylrutin is preferably used, and a composition containing α-glucosylrutin that is any of the first enzyme-treated rutin and the second enzyme-treated rutin may be used.

When, for example, the effect of the present invention is taken into account, the enzyme-treated rutin is preferably a mixture containing at least α-glucosylrutin and further containing isoquercitrin.

Such a mixture can be produced by the following procedures: (i) the above-described first enzyme-treated rutin is prepared, (ii) the first enzyme-treated rutin is treated with an enzyme having a glucoamylase activity and almost all α-glucosylrutin is converted to α-monoglucosylrutin, and (iii) the first enzyme-treated rutin is treated with an enzyme having a rhamnosidase activity at the same time and almost all unreacted rutin is converted to isoquercitrin.

Examples of the commercially available product of the enzyme-treated rutin include products "αG rutin PS", "αG rutin P" and "αG rutin H" manufactured by Toyo Sugar Refining Co., Ltd. "αG rutin PS" is a composition containing 75 mass% of α-monoglucosylrutin and 15 mass% of isoquercitrin. In addition, "αG rutin P" is a composition containing 60 mass% of α-glucosylrutin, 10 mass% of rutin and 1% of isoquercitrin.

As α-glucosylrutin, α-monoglucosylrutin is preferable. This is because the molecular weight of α-monoglucosylrutin is smaller than the molecular weight of α-polyglucosylrutin, and thus the number of molecules per unit mass becomes larger in α-monoglucosylrutin, which is considered to be advantageous in terms of the action and effect.

The presence of a variety of α-glucosylrutin and other components that are contained in the enzyme-treated rutin can be confirmed by HPLC chromatogram, and the contents of the individual components and the purity of a desired specific component can be calculated from the peak area of the chromatogram.

The method for producing α-glucosylrutin is not particularly limited, and a well-known method can be used. As described above, α-glucosylrutin is preferably produced by enzyme treatment of rutin since the yield is favorable and the production is easy. The method for procuring and preparing rutin is not particularly limited, and a compound that is ordinarily produced and sold as a reagent or a purified substance may be used or a compound prepared by extraction from a raw material such as the external skins of citrus fruits (such as tangerines and oranges) or buckwheat's seeds may be used.

<α-Glucosyl Hesperidin>

α-Glucosyl hesperidin (also referred to as α glucosyl hesperidin) is a collective term of compounds in which one or more molecules of glucose are added to a hydroxyl group in the rutinose unit of hesperidin by an α-1,4 linkage. α-Glucosyl hesperidin in the present invention may be composed only of one compound having such a structure or may be a mixture of two or more compounds having such a structure. Hesperidin is a compound in which β-rutinose (6-O-α-L-rhamnosyl-β-D-glucose) is linked to a hydroxyl group at the position 7 of hesperetin, that is, a hesperetin glycoside.

α-Glucosyl hesperidin can be represented by the following formula (2). In the formula (2), n is 0 or an integer of 1 or more, for example, an integer of 1 to 19.

[Chem. 2]

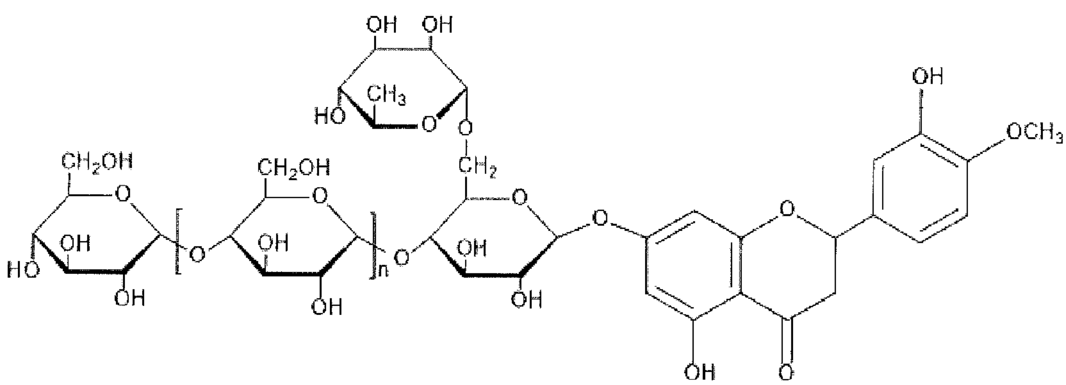

・・・ (2)

α-Glucosyl hesperidin is a compound that is contained as a main component in a material known as "enzyme-treated hesperidin" (also called "transglycosylated hesperidin" in some cases). α-Glucosyl hesperidin to which only one

molecule of glucose is linked is called "α-monoglucosyl hesperidin", and α-glucosyl hesperidin to which two or more molecules of glucose are linked is called "α-polyglucosyl hesperidin". That is, in the formula (2), α-monoglucosyl hesperidin is a compound for which n is 0, and α-polyglucosyl hesperidin is ordinarily a compound for which n is 1 to 19.

The enzyme-treated hesperidin is an aggregate of compounds formed by enzyme treatment of sugar of hesperidin and normally contains a mixture of compounds in which the number of glucose molecules linked to hesperidin is different from one another, for example, a mixture composed of α-monoglucosyl hesperidin and α-polyglucosyl hesperidin. In addition, the enzyme-treated hesperidin may contain not only α-glucosyl hesperidin but also an unreacted hesperidin and a hesperidin derivative other than α-glucosyl hesperidin such as 7-glucosyl hesperetin (also referred to as the different hesperidin derivative). However, the enzyme-treated hesperidin preferably does not contain hesperetin.

Examples of the enzyme treatment of the sugar of hesperidin are as described below. (1) A glycosyltransferase is caused to act on hesperidin in the coexistence of a glycosyl donor to add glucose to the glucose unit of hesperidin by an α-1,4 linkage, whereby α-glucosyl hesperidin is formed, and a composition containing unreacted hesperidin and α-glucosyl hesperidin is obtained (first enzyme-treated hesperidin). (2) Glucoamylase, for example, is caused to act on the α-glucosyl hesperidin formed by (1), and all glucose molecules other than one molecule from glucose chains linked to the glucose unit of hesperidin are cut, whereby α-monoglucosyl hesperidin is formed, and a composition containing hesperidin remaining unreacted and α-monoglucosyl hesperidin is obtained (second enzyme-treated hesperidin). (3) α-L-rhamnosidase is caused to act on the hesperidin remaining unreacted in (2), rhamnose that is contained in the rutinose unit of rutin is cut to form 7-glucosyl hesperetin, and a composition containing 7-glucosyl hesperetin and α-monoglucosyl hesperidin is obtained (third enzyme-treated hesperidin).

Examples of the first enzyme treatment include treatment in which a glycosyltransferase (for example, an enzyme having a function of adding glucose to hesperidin such as cyclodextrin glucanotransferase (CGTase, EC2.4.1.19)) is caused to act on hesperidin in the coexistence of an α-glucosyl sugar compound (for example, cyclodextrin, partially degraded starch).

In the collagenase activity inhibitor of the present invention, when, for example, the effect of the present invention is taken into account, enzyme-treated hesperidin that is a composition containing α-glucosyl hesperidin is preferably used, and a composition of any of the first enzyme-treated hesperidin, the second enzyme-treated hesperidin and the third enzyme-treated hesperidin may be used.

When the effect of the present invention is taken into account, the enzyme-treated hesperidin is preferably a mixture containing at least α-glucosyl hesperidin and further containing any one or both of hesperidin and 7-glucosyl hesperetin.

Examples of the commercially available product of the enzyme-treated hesperidin include a product "αG hesperidin PS-CC" manufactured by Toyo Sugar Refining Co., Ltd. The "αG hesperidin PS-CC" contains 80 mass% or more of α-monoglucosyl hesperidin and contains hesperidin and 7-glucosyl hesperetin.

As α-glucosyl hesperidin, α-monoglucosyl hesperidin is preferable. This is because the molecular weight of α-monoglucosyl hesperidin is smaller than the molecular weight of α-polyglucosyl hesperidin, and thus the number of molecules per unit mass becomes larger in α-monoglucosyl hesperidin, which is considered to be advantageous in terms of the action and effect.

The α-monoglucosyl hesperidin can be produced by causing a glycohydrolase to act on α-polyglucosyl hesperidin and cutting the glucose molecules linked to hesperidin except only one glucose molecule (second enzyme-treated hesperidin). As the glycohydrolase, an enzyme having a glucoamylase activity that cuts an α-1,4-glucoside linkage by glucose unit, for example, glucoamylase (EC3.2.1.3) is exemplified. The proportion of the α-monoglucosyl hesperidin in the α-glucosyl hesperidin can be adjusted according to conditions such as the temperature or time conditions of the enzyme treatment using glucoamylase, and furthermore, a method in which α-monoglucosyl hesperidin is purified and collected from the mixture as the enzyme-treated hesperidin is also well known.

The presence of α-glucosyl hesperidin, hesperidin and other components that are contained in the enzyme-treated hesperidin can be confirmed by HPLC chromatogram, and the contents of the individual components and the purity of a desired specific component can be calculated from the peak area of the chromatogram.

The method for producing α-glucosyl hesperidin is not particularly limited, and a well-known method can be used. α-Glucosyl hesperidin is preferably produced by the above-described enzyme treatment of hesperidin since the yield is favorable and the production is easy. The method for procuring and preparing hesperidin is not particularly limited, and a compound that is ordinarily produced and sold as a reagent or a purified substance may be used or a compound prepared by extraction from a raw material such as the external skins of citrus fruits (such as tangerines and oranges) may be used.

<α-Glucosyl Naringin>

α-Glucosyl naringin (also referred to as α glucosyl naringin) is a collective term of compounds in which one or more molecules of glucose are added to a hydroxyl group in naringin. Naringin is one of flavonoids having a structure

in which neohesperidose (L-rhamnosyl-(α1→2)-D-glucose) is β-linked to a hydroxyl group at the position 7 of a naringenin (5,7,4'-trihydroxyflavanone) skeleton. α-Glucosyl naringin has a structure in which one or more molecules of α-glucose are linked to at least one of a hydroxyl group at the position 3 (position 3") of a glucose residue in a neohesperidose residue and a hydroxyl group at the position 4 (position 4') of a phenyl group in a naringenin skeleton, which are in the naringin. α-Glucosyl naringin in the present invention may be composed only of one compound having such a structure or may be a mixture of two or more compounds having such a structure.

α-Glucosyl naringin can be represented by the following formula (3). In the formula (3), m in $R^1$ and n in $R^2$ each mean the number of α-glucose residues linked to the position 3" or the position 4' and each independently represent 0 or more and, normally, an integer of 25 or less. Here, in order for the formula (3) to represent "a-glucosyl naringin", it is necessary that $m + n \geq 1$ is satisfied, that is, at least one molecule of α-glucose is linked to naringin (in the case of $m = n = 0$, that is, in a case where both $R^1$ and $R^2$ are -H, the formula (3) represents "naringin").

[Chem. 3]

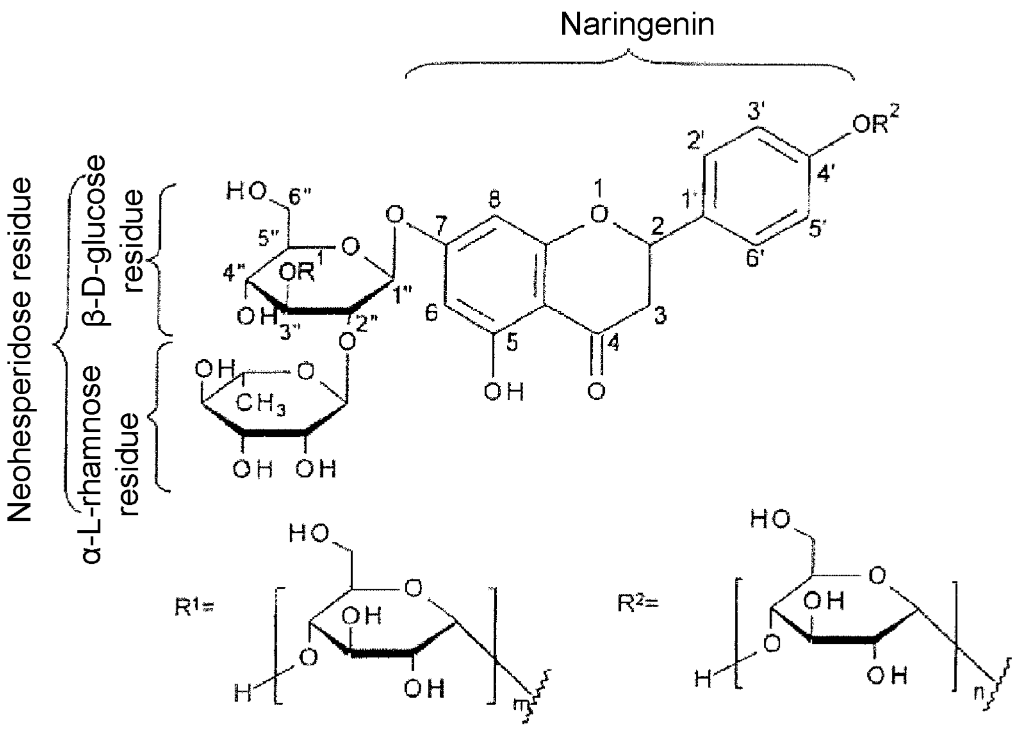

· · · (3)

α-Glucosyl naringin is a compound that is contained as a main component in a material known as "enzyme-treated naringin" (also called "transglycosylated naringin" in some cases). α-Glucosyl naringin to which only one molecule of glucose is linked is called "α-monoglucosyl naringin", and α-glucosyl naringin to which two or more molecules of glucose are linked is called "α-polyglucosyl naringin".

The enzyme-treated naringin is, for example, a product that is obtained by reacting a glycosyltransferase (for example, cyclodextrin glucosyltransferase) with a mixture of naringin and a glycosyl donor (for example, dextrin) (called "first enzyme-treated naringin" in the present specification) and is an aggregate of a variety of compounds in which one or more molecules of glucose are added to a hydroxyl group in naringin. Therefore, the enzyme-treated naringin normally contains a mixture of compounds in which the number of glucose molecules linked to naringin is different from one another, for example, a mixture composed of α-monoglucosyl naringin and α-polyglucosyl naringin. In addition, the enzyme-treated naringin may contain, in addition to α-glucosyl naringin, unreacted naringin and a naringin derivative other than α-glucosyl naringin such as 7-glucosyl naringenin (referred to as the different naringin derivative).

Regarding the basic method for producing the first enzyme-treated naringin, for example, JPH4-13691 A can be referred to. If necessary, the first enzyme-treated naringin is purified using, for example, a porous synthetic adsorbent and an appropriate eluent, whereby a first enzyme-treated naringin in which the glycosyl donor and other impurities are removed, furthermore, the content of naringin is decreased and the purity of α-glucosyl naringin is increased (α-glucosyl

naringin purified substance) can be obtained.

As α-glucosyl naringin, a substance in which one molecule of α-glucose is linked to the position 3" of naringin and/or one molecule of α-glucose is linked to the position 4' of naringin, that is, at least one α-glucosyl naringin selected from 3"-α-monoglucosyl naringin (in the formula (3), m = 1 and n = 0), 4'-α-monoglucosyl naringin (in the formula (3), m = 0 and n = 1) and 3"-4'-α-diglucosyl naringin (in the formula (3), m = 1 and n = 1) is preferable, and, particularly, 3"-α-monoglucosyl naringin is more preferable. This is because the molecular weight of α-monoglucosyl naringin is smaller than the molecular weight of α-polyglucosyl naringin, and thus the number of molecules per unit mass becomes larger in α-monoglucosyl naringin, which is considered to be advantageous in terms of the action and effect.

Enzyme-treated naringin containing a large amount of the above-described three kinds of α-mono/diglucosyl naringin (called "second enzyme-treated naringin" in the present specification) can be obtained by, for example, treating the above-described first enzyme-treated naringin with an enzyme having a glucoamylase activity and cutting sugar chains which have been transferred to the position 3" and/or the position 4' of naringin by the glycosyltransferase and to which two or more molecules of α-glucose are linked by α-1,4 linkages except only α-glucose residues as many as one molecule of the base. Furthermore, the second enzyme-treated naringin is treated with an enzyme having an α-glucosidase activity, and α-glucose residues as many as one molecule directly linked to the hydroxyl group at the position 4' are cut, whereby an enzyme-treated naringin in which 3"-α-monoglucosyl naringin is left and 4'-α-monoglucosyl naringin and 3"-4'-α-diglucosyl naringin are rarely or not contained (called "third enzyme-treated naringin" in the present specification) can be obtained. Regarding the basic method for producing the second and third enzyme-treated naringin, for example, JP2002-199896A can be referred to.

In addition, when transglucosidase is caused to act on the first enzyme-treated naringin, since transglucosidase is an enzyme having both a glucoamylase activity and an α-glucosidase activity, the third enzyme-treated naringin containing abundant 3"-α-monoglucosyl naringin can be obtained in one step rather than two-step treatment as described above. Furthermore, if necessary, treatment in which an enzyme having a β-glucosidase activity is caused to act on the third enzyme-treated naringin (may be caused to act on the first enzyme-treated naringin simultaneously with transglucosidase) to cut a neohesperidose residue which is in unreacted naringin that has been dissolved in an aqueous solution in a small amount (and in which a sugar chain has not been modified by the glycosyltransferase) from naringenin, which is an aglycone of the neohesperidose residue may be carried out. Naringenin that is formed by such treatment has a lower solubility than naringin and is thus precipitated and can be easily removed from the aqueous solution, which makes it possible to collect 3"-α-monoglucosyl naringin from the aqueous solution with a higher purity.

In the collagenase activity inhibitor of the present invention, when, for example, the effect of the present invention is taken into account, enzyme-treated naringin that is a composition containing α-glucosyl naringin is preferably used, and a composition of any of the first enzyme-treated naringin, the second enzyme-treated naringin and the third enzyme-treated naringin may be used.

When, for example, the effect of the present invention is taken into account, the enzyme-treated naringin is preferably a mixture containing at least α-glucosyl naringin and further containing any one or both of naringin and 7-glucosyl naringenin.

The presence of α-glucosyl naringin, naringin and other components that are contained in the enzyme-treated naringin can be confirmed by HPLC chromatogram, and the contents of the individual components and the purity of a desired specific component can be calculated from the peak area of the chromatogram.

The method for producing α-glucosyl naringin is not particularly limited, and a well-known method can be used. α-Glucosyl naringin is preferably produced by the above-described enzyme treatment of naringin since the yield is favorable and the production is easy. The method for procuring and preparing naringin is not particularly limited, and a compound that is ordinarily produced and sold as a reagent or a purified substance may be used or a compound prepared by extraction from a raw material such as the external skins of citrus fruits (such as Chinese citrons and grapefruits) may be used.

<Collagenase Activity>

A collagenase is a protease that specifically cuts collagen. As collagen, a variety of molecular species such as Type I, Type II, Type III, Type IV, Type V, Type VI, Type VII, Type VIII, Type IX, Type X, Type XI, Type XII and Type XIII have been reported, and, in the present specification, the term "collagen" is used to include all of these molecular species. In addition, animal species from which collagen is derived are also not particularly limited.

In the present specification, the collagenase activity means an activity for decomposition of at least one of all of these molecular species of collagen and may be an activity for decomposition of only one species of collagen molecules or may be an activity for decomposition of two or more arbitrarily-selected species of collagen molecules. The collagenase activity is preferably an activity for decomposition of at least one species of collagen selected from Type I collagen, Type II collagen, Type III collagen and Type IV collagen, more preferably an activity for decomposition of at least one species of collagen selected from Type I collagen and Type II collagen and still more preferably an activity for decomposition of

at least Type I collagen.

A method for measuring the collagenase activity is not particularly limited, and a well-known method can be used. Examples thereof include a method in which the fluorescence intensity of a decomposition product is measured using fluorescent collagen that emits fluorescence when cut by collagenase, a method in which the radiation dose of a decomposition product is measured using collagen labeled with a radioisotope, and a colorimetric determination method of a decomposition product in which a collagen-like synthetic substrate (such as FALGPA) is used. The method in which the fluorescence intensity of a decomposition product is measured using fluorescent collagen that emits fluorescence when cut by collagenase is preferable since the operation is simple.

<Collagenase Activity Inhibitor>

The collagenase activity inhibitor needs to have an action of inhibiting the collagenase activity, and the inhibition rate is not particularly limited. The collagenase activity inhibition rate is, for example, 10% or more, 20% or more, 30% or more, 40% or more, 50% or more, 60% or more, 70% or more, 80% or more, or 90% or more.

The collagenase activity inhibitor is preferably a collagenase activity inhibitor that inhibits an activity for decomposition of Type I collagen. In a case where the collagenase activity inhibitor is a collagenase activity inhibitor that inhibits an activity for decomposition of Type I collagen, the collagenase activity inhibitor may inhibit an activity for decomposition of other collagen at the same time.

The collagenase activity inhibitor needs to contain at least one component (A) selected from α-glucosylrutin, α-glucosyl hesperidin and α-glucosyl naringin and may be composed only of the component (A) or may further contain a well-known arbitrary component such as an excipient, a stabilizer, a wetting agent or an emulsifier as long as the collagenase activity inhibition effect of the component (A) is not hindered. In addition, the collagenase activity inhibitor may be at least one selected from enzyme-treated rutin, enzyme-treated hesperidin and enzyme-treated naringin, which are compositions containing the component (A), or may be a substance containing at least one selected from enzyme-treated rutin, enzyme-treated hesperidin and enzyme-treated naringin.

Examples of the collagenase activity inhibitor containing the component (A) include commercially available products such as products "αG rutin PS", "αG rutin P", "αG rutin H" and "αG hesperidin PS-CC" manufactured by Toyo Sugar Refining Co., Ltd. These products themselves may be used as collagenase activity inhibitors or compositions obtained by blending these products may be used as collagenase activity inhibitors.

An aspect of the present invention is a collagenase activity inhibitor containing at least one component (A) selected from α-glucosylrutin, α-glucosyl hesperidin and α-glucosyl naringin, in which the component (A) contains at least one selected from α-monoglucosylrutin, α-monoglucosyl hesperidin and α-monoglucosyl naringin, and the collagenase activity inhibitor inhibits an activity for decomposition of Type I collagen.

An aspect of the present invention is a collagenase activity inhibitor containing at least one component (A) selected from α-glucosylrutin, α-glucosyl hesperidin and α-glucosyl naringin, in which the content of the component (A) is 70 mass% or more, and the collagenase activity inhibitor inhibits an activity for decomposition of Type I collagen.

An aspect of the present invention is a collagenase activity inhibitor containing at least one component (A) selected from α-glucosylrutin, α-glucosyl hesperidin and α-glucosyl naringin, in which the component (A) contains at least one selected from α-monoglucosylrutin, α-monoglucosyl hesperidin and α-monoglucosyl naringin, the content of the component (A) is 70 mass% or more, and the collagenase activity inhibitor inhibits an activity for decomposition of Type I collagen.

<Uses of Collagenase Activity Inhibitor>

The uses of the collagenase activity inhibitor are not particularly limited, and the collagenase activity inhibitor of the present invention has a strong action of inhibiting the activity of collagenase that cuts collagen and thus can be administered to living bodies once or a plurality of times for the purpose of inhibiting the collagenase activity increased in the living bodies or for the purpose of suppressing a decrease in the amount of collagen in the living bodies, for example.

The collagenase activity inhibitor is useful for the prevention or improvement of a disease or a symptom in which the collagenase activity is involved, and contributes to the prevention or improvement of the onset and progress of articular rheumatism, osteoarthritis, osteoporosis, cancer metastasis, development of ulcer, periodontitis or cavity formation, for example. In addition, the collagenase activity inhibitor contributes to skin elasticity or tension and the prevention or improvement of wrinkles or sagging, that is, the prevention of skin aging.

The amount of the collagenase activity inhibitor to be administered needs to be appropriately selected depending on the kind of a disease or a symptom or the degree of a symptom in which the collagenase activity is involved. For example, in a case where the collagenase activity inhibitor is orally administered, from the viewpoint of the collagenase activity inhibition effect, the amount of the component (A) to be administered per day is preferably 10 mg to 700 mg and more preferably 100 mg to 500 mg. For example, in a case where the collagenase activity inhibitor is externally applied,

from the viewpoint of the collagenase activity inhibition effect, the amount of the component (A) to be administered per day is preferably 1 mg to 100 mg and more preferably 40 mg to 80 mg. In addition, the above-described amount to be administered per day can be divided, for example, into one to three doses per day for administration, and is preferably divided into two or three doses for administration.

The duration of administration of the collagenase activity inhibitor needs to be appropriately selected depending on the kind of a disease or the degree of a symptom in which the collagenase activity is involved. From the viewpoint of the collagenase activity inhibition effect, the duration of administration is preferably 7 to 80 days and more preferably 14 days to 60 days.

<Preparation Containing Collagenase Activity Inhibitor>

The collagenase activity inhibitor may be administered to living bodies as it is or may be administered as a preparation produced by blending an effective amount of the collagenase activity inhibitor with a pharmaceutically acceptable carrier. Examples of the preparation include food and drink, a medicine, a quasi-drug, a cosmetic product, animal feed and pet food, and food and drink, a medicine, a quasi-drug or a cosmetic product is preferable. An administration method is not particularly limited, and the collagenase activity inhibitor can be orally or parenterally administered. The above-described preparations are preferably administered parenterally in view of easy administration.

In the case of oral administration, the preparation may be food and drink (including foods with health claims such as foods for specified health use, foods with nutritional claims and foods with functional claims or other so-called health foods and supplements), a medicine, a quasi-drug, animal feed or pet food, for example.

Oral preparations can be a solid form or a liquid form (including a paste form). Dosage forms are not limited, and specific examples of solid preparations include a powder, a granule, a tablet, a capsule and a pastille. In addition, examples of liquid preparations include an internal liquid, a suspension, an emulsion, a syrup and a drink, and these dosage forms or other dosage forms are appropriately selected depending on the purposes. The dosage form is preferably a tablet or a capsule since the administration is easy and the collagenase activity inhibition effect is easily developed. In solid preparations, an auxiliary agent such as an excipient, a binder, a disintegrant, a lubricant, a flavoring agent or a stabilizer may be used.

In the case of parenteral administration, the preparations can be, for example, an injection, an external skin preparation or a suppository and are preferably an external skin preparation including a medicine, a quasi-drug and a cosmetic product in order to effectively develop the collagenase activity inhibition effect.

The form and dosage form of the skin external preparation are not particularly limited and are normally a liquid form (including a paste form and a gel form). Specific examples thereof include a liquid external preparation, a suspension, an emulsion, cream and ointment. These dosage forms or other dosage forms are appropriately selected depending on the purposes. Among them, a liquid external preparation, an emulsion, cream or ointment is preferable since the administration is easy and the collagenase activity inhibition effect is easily developed.

Preparations containing the collagenase activity inhibitor can be produced by adding the collagenase activity inhibitor according to a method that is ordinarily used for these preparations. The collagenase activity inhibitor may be added in the initial stage of the production step of the preparation or may be added in the middle stage or final stage of the production step, and, as the addition method, an appropriate method may be selected from mixing, kneading, dissolution, immersion, scattering, spraying and application, for example, depending on the form of the preparation.

The component (A), which is an active component of the collagenase activity inhibitor, has favorable water solubility and thus can be uniformly dissolved or dispersed even when added to water or preparations containing a large amount of moisture.

The amount of the collagenase activity inhibitor to be blended with a preparation needs to be appropriately selected depending on the kind of a disease or the degree of a symptom in which the collagenase activity is involved, and, from the viewpoint of easy administration or stability in preparations, the amount of the component (A) to be blended is preferably 0.05 to 2.0 mass% and more preferably 0.25 to 1.0 mass%.

## Examples

Next, the present invention will be described in more detail by showing Examples, but the present invention is not limited to these Examples.

[Example 1]

The activity of collagenase was measured using a Type I collagenase assay kit (manufactured by Cosmo Bio Co., Ltd, model No. AK37, composed of fluorescence-labeled collagen, a buffer fluid A and a buffer fluid B). The specific procedure is as described below.

(Preparation of Reagent)

A necessary amount of fluorescence-labeled collagen and the same amount of the buffer fluid A were mixed together to prepare a substrate liquid, and the substrate liquid was shielded from light with aluminum foil and stored on ice until use. In order to prevent fluorescence quenching, the substrate liquid was prepared at the time of use.

A necessary amount of the buffer fluid A and the same amount of ion exchanged water were mixed together to prepare a buffer solution, and the buffer solution was stored on ice until use.

4 mg of collagenase (manufactured by MP Biomedicals, LLC., model No. 195109) was dissolved (1000 U/mL) in 0.5 mL of the buffer solution to prepare a collagenase undiluted solution, and this was diluted with the buffer solution to prepare a collagenase solution.

As samples, the following αG rutin was used as α-glucosylrutin, the following αG hesperidin was used as α-glucosyl hesperidin, and the following αG naringin was used as α-glucosyl naringin. The composition of each sample is as described below.

αG Rutin: 71 mass% of α-monoglucosylrutin and 14 mass% of isoquercitrin
αG Hesperidin: 85 mass% of α-monoglucosyl hesperidin and 10 mass% of 7-glucosyl hesperetin
αG Naringin: 81 mass% of α-monoglucosyl naringin and 12 mass% of 7-glucosyl naringenin
600 mg of each sample was adjusted to a constant volume of 5 mL with ion exchanged water to prepare a sample undiluted solution. This sample undiluted solution was diluted with ion exchanged water stepwise until each final concentration was reached, thereby preparing each sample solution (Table 1).

[Table 1]

Table 1 Preparation of sample solution and concentration of collagenase

| Sample name | Final concentration (%) of sample upon analysis | | | | | | | Concentration (μg/mL) of collagenase used |
|---|---|---|---|---|---|---|---|---|
| αG rutin | 0.05 | 0.1 | 0.25 | 0.5 | 1 | | | 80 |
| αG hesperidin | 0.05 | 0.1 | 0.25 | 0.5 | 1 | 2 | 3 | |
| αG naringin | 0.05 | 0.1 | 0.25 | 0.5 | 1 | 3 | | |

(Reaction and Activity Measurement)

1. 50 μL of the substrate liquid was added to each 1.5 mL tube. In addition to a tube for each sample, tubes for control, blank for control and blank for each sample were also prepared. In a case where a sample itself has color, when the sample is measured as it is, there are cases where, compared with control (to which the sample is not added), the measurement value of the fluorescence intensity becomes high by the color of the sample. In order to correct this deviation in the measurement value, blank for subtracting the fluorescence intensity value attributed to the color of the sample was made. That is, blank was made by adding the sample, but not adding a collagenase solution to be described below, and this was used as the blank for sample. The experiment was carried out three times under one experiment condition.
2. 25 μL of each sample solution was added to 1. and stirred. To the control and the blank for control, ion exchanged water was added instead of the sample solution.
3. 25 μL of the collagenase solution was added to 2., stirred and reacted at 35°C for 30 minutes. To the blank for sample and the blank for control, the collagenase solution was not added, but the buffer solution was added.
4. 300 μL of the buffer fluid B was added to all of the tubes, intensively stirred and left to stand in an ice bath for 15 minutes.
5. Centrifugal separation (4°C, 4000 rpm, 10 minutes) was carried out, and 100 μL of supernatant was added onto a fluorescence black 96-well plate.
6. The fluorescence intensity at an excitation wavelength of 495 nm and a fluorescent wavelength of 520 nm was measured with a microplate reader.
7. The collagenase activity inhibition rate (%) was calculated from a formula I.
[Math. 1]

$$\text{Collagenase activity inhibition rate (\%)} = \{(C-C') - (S-S')\} / (C-C') \times 100 \quad \text{(Formula I)}$$

In the formula I,

C is the fluorescence intensity value of control,
C' is the fluorescence intensity value of blank for control,
S is the fluorescence intensity value of sample and
S' is the fluorescence intensity value of blank for sample.

(Results)

The results are shown in Figure 1. The inhibition rates of the collagenase activity increased with the concentrations of αG rutin, αG hesperidin and αG naringin. It was clarified that α-glucosylrutin, α-glucosyl hesperidin and α-glucosyl naringin had a collagenase activity inhibition effect. For αG rutin, αG hesperidin and αG naringin, collagenase activity inhibition was observed at a concentration of 0.05% or more, high collagenase activity inhibition was observed at a concentration of 0.1% or more, and higher collagenase activity inhibition was observed at a concentration of 0.25% or more. In addition, for α-glucosyl hesperidin and α-glucosyl naringin, there was a tendency that the collagenase activity inhibition effect was strong at relatively low concentrations, particularly, at 0.05% and 0.1%.

[Example 2]

The collagenase activity was measured in the same manner as in Example 1 except that 2 M tris-hydrochloric acid buffer fluid (pH: 8.0) was used instead of ion exchanged water in the preparation and dilution of the sample solution.
As samples, as in Example 1, αG rutin, αG hesperidin and αG naringin were used.

[Comparative Example 1]

As a comparative sample, vitamin C (ascorbic acid, manufactured by Nacalai Tesque, Inc., No. 03420-65) was used. The prepared sample solutions are as shown in Table 2.

[Table 2]

Table 2 Preparation of sample solution and concentration of collagenase

| Sample name | Final concentration (%) of sample upon analysis | | | Concentration (μg/mL) of collagenase used |
|---|---|---|---|---|
| Vitamin C | 0.1 | 0.25 | 0.5 | 80 |
| αG rutin | 0.1 | 0.25 | 0.5 | |
| αG hesperidin | 0.1 | 0.25 | 0.5 | |
| αG naringin | 0.1 | 0.25 | 0.5 | |

(Results)

The results are shown in Figure 2. The collagenase activity inhibition rates of αG rutin, αG hesperidin and αG naringin were high compared with that of the vitamin C at the same concentration. It was clarified that α-glucosylrutin, α-glucosyl hesperidin and α-glucosyl naringin, which were the derivatives of rutin, hesperidin and naringin, respectively and also called vitamin P, had a stronger collagenase activity inhibition effect than vitamin C.

[Example 3]

The collagenase activity was measured in the same manner as in Example 1 except the following two points.

1. The collagenase activity was measured using 1 M tris-hydrochloric acid buffer fluid (pH: 6.5) containing 20 mM calcium chloride instead of ion exchanged water in the preparation and dilution of the sample solution.
2. 3 mg of collagenase Type I (manufactured by Worthington Biochemical Corporation, No. LS004194, 175 U/mg) was dissolved (1050 U/mL) in 0.5 mL of the buffer solution to prepare a collagenase undiluted solution.

As samples, as in Example 1, αG rutin, αG hesperidin and αG naringin were used.

[Comparative Example 2]

As a comparative sample, vitamin C (sodium ascorbate, manufactured by Nacalai Tesque, Inc., No. 03422-45) was used. The prepared sample solutions are as shown in Table 3.

[Table 3]

Table 3 Preparation of sample solution and concentration of collagenase

| Sample name | Final concentration (%) of sample upon analysis | | | | | | Concentration (μg/mL) of collagenase used |
|---|---|---|---|---|---|---|---|
| Sodium ascorbate* | 0.1 | 0.25 | 0.5 | 1.0 | 2.0 | 3.0 | 60 |
| αG rutin | | | | | | | |
| αG hesperidin | | | | | | | |
| αG naringin | | | | | | | |
| * As the sample final concentration at the time of analyzing sodium ascorbate in Table 3, a calculated ascorbic acid-equivalent concentration was recorded. In the actual test, sodium ascorbate having a concentration 1.125 times the concentration in Table 3 was used. | | | | | | | |

(Results)

The results are shown in Figure 3. Even at pH of 6.5, the collagenase activity inhibition rates of αG rutin, αG hesperidin and αG naringin were high compared with that of the vitamin C at the same concentration. It was clarified that, even at pH of 6.5, α-glucosylrutin, α-glucosyl hesperidin and α-glucosyl naringin, which were the derivatives of rutin, hesperidin and naringin, respectively and also called vitamin P, had a stronger collagenase activity inhibition effect than vitamin C.

Industrial Applicability

According to the present invention, it is possible to provide a collagenase activity inhibitor exhibiting an excellent collagenase activity inhibition action.

## Claims

1. A collagenase activity inhibitor comprising at least one component (A) selected from α-glucosylrutin, α-glucosyl hesperidin and α-glucosyl naringin.

2. The collagenase activity inhibitor according to claim 1, wherein the component (A) comprises at least one selected from α-monoglucosylrutin, α-monoglucosyl hesperidin and α-monoglucosyl naringin.

3. The collagenase activity inhibitor according to claim 1, wherein a content of the component (A) is 70 mass% or more.

4. The collagenase activity inhibitor according to claim 2, wherein a content of the component (A) is 70 mass% or more.

5. The collagenase activity inhibitor according to claim 1, wherein the collagenase activity inhibitor inhibits an activity for decomposition of Type I collagen.

[Fig. 1]

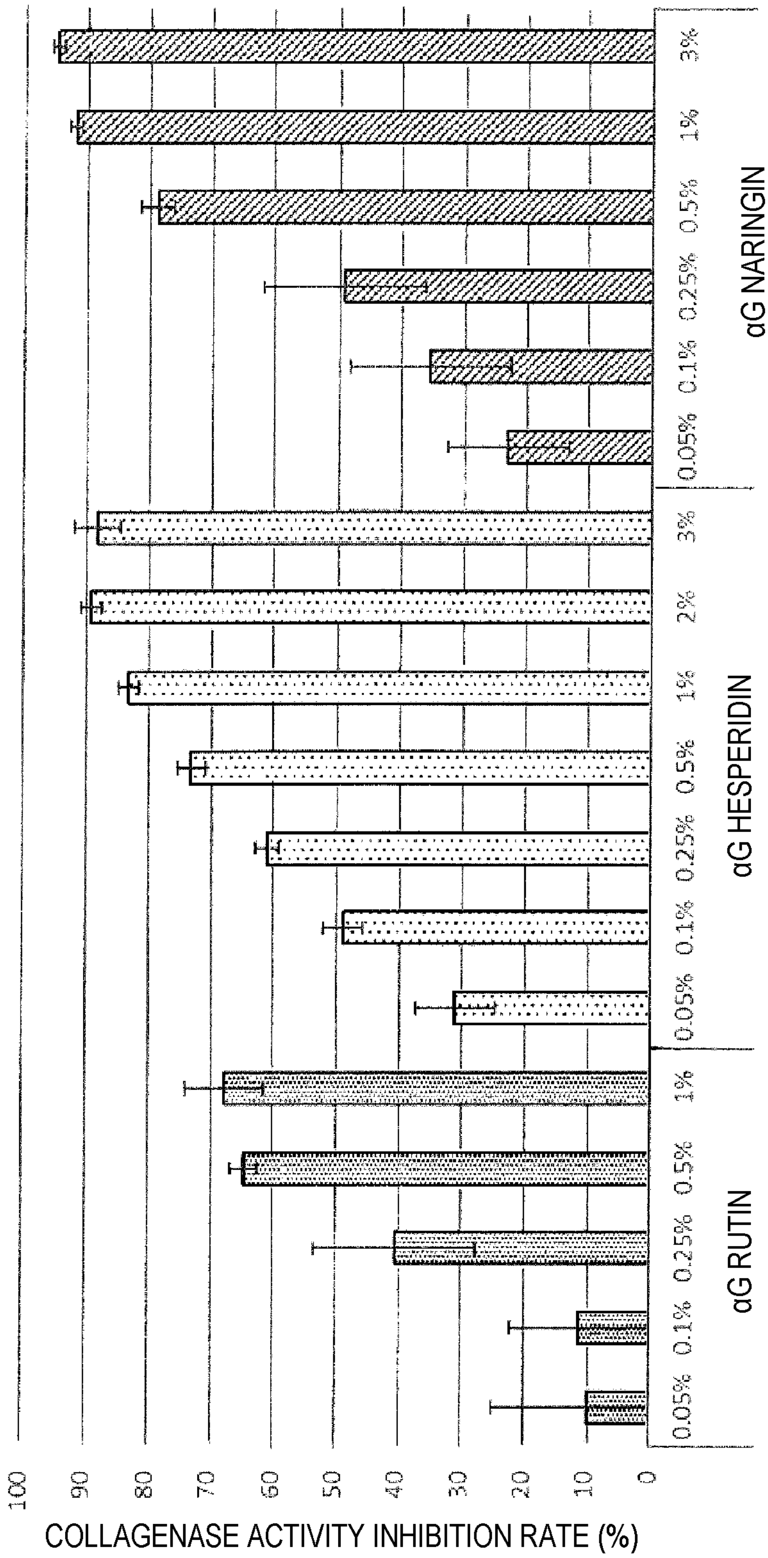
αG NARINGIN
3%
1%
0.5%
0.25%
0.1%
0.05%
αG HESPERIDIN
3%
2%
1%
0.5%
0.25%
0.1%
0.05%
αG RUTIN
1%
0.5%
0.25%
0.1%
0.05%
100
90
80
70
60
50
40
30
20
10
0
COLLAGENASE ACTIVITY INHIBITION RATE (%)

[Fig. 2]

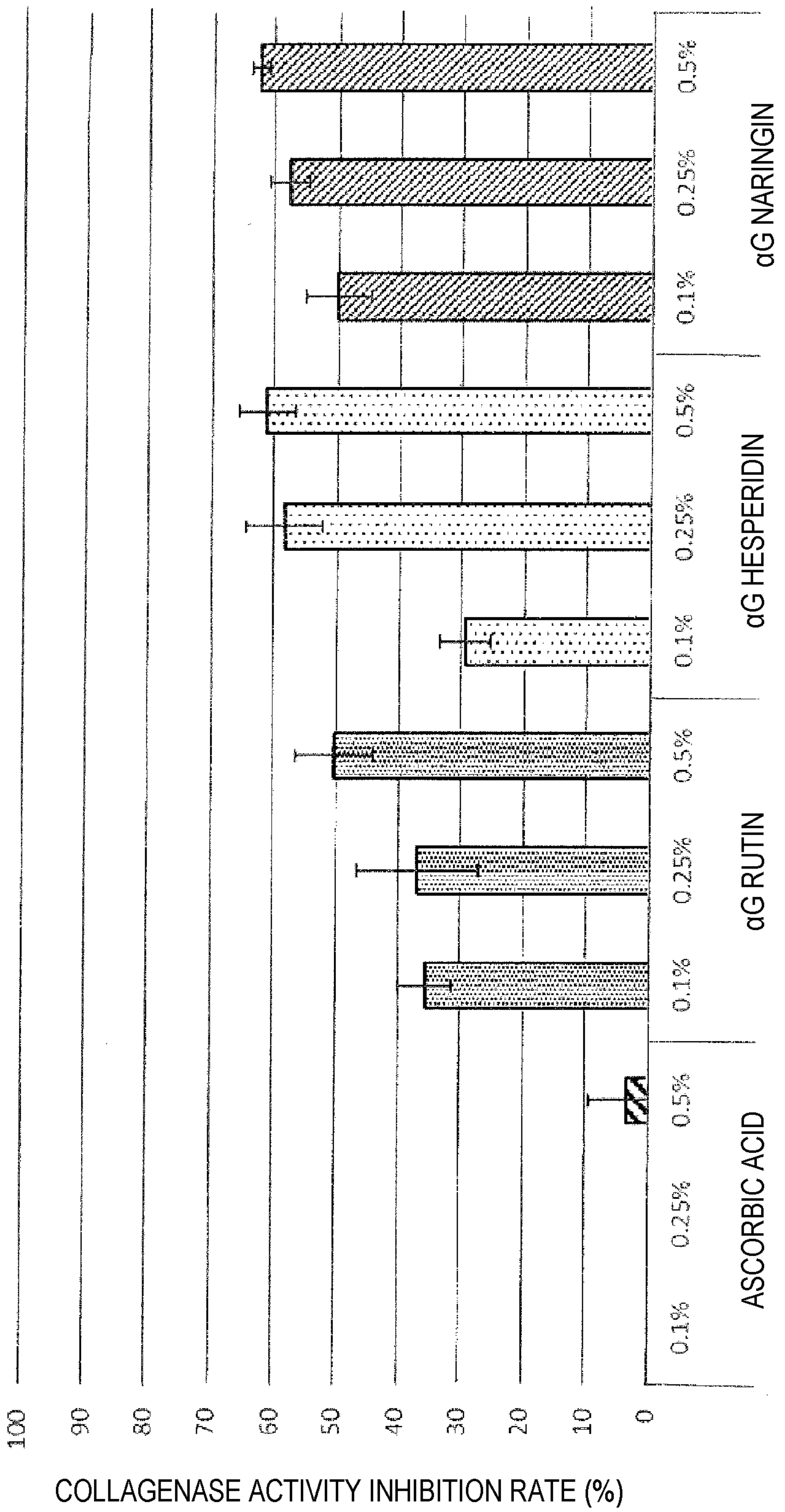
COLLAGENASE ACTIVITY INHIBITION RATE (%)
0
10
20
30
40
50
60
70
80
90
100
ASCORBIC ACID
αG RUTIN
αG HESPERIDIN
αG NARINGIN
0.1%
0.25%
0.5%

[Fig. 3]

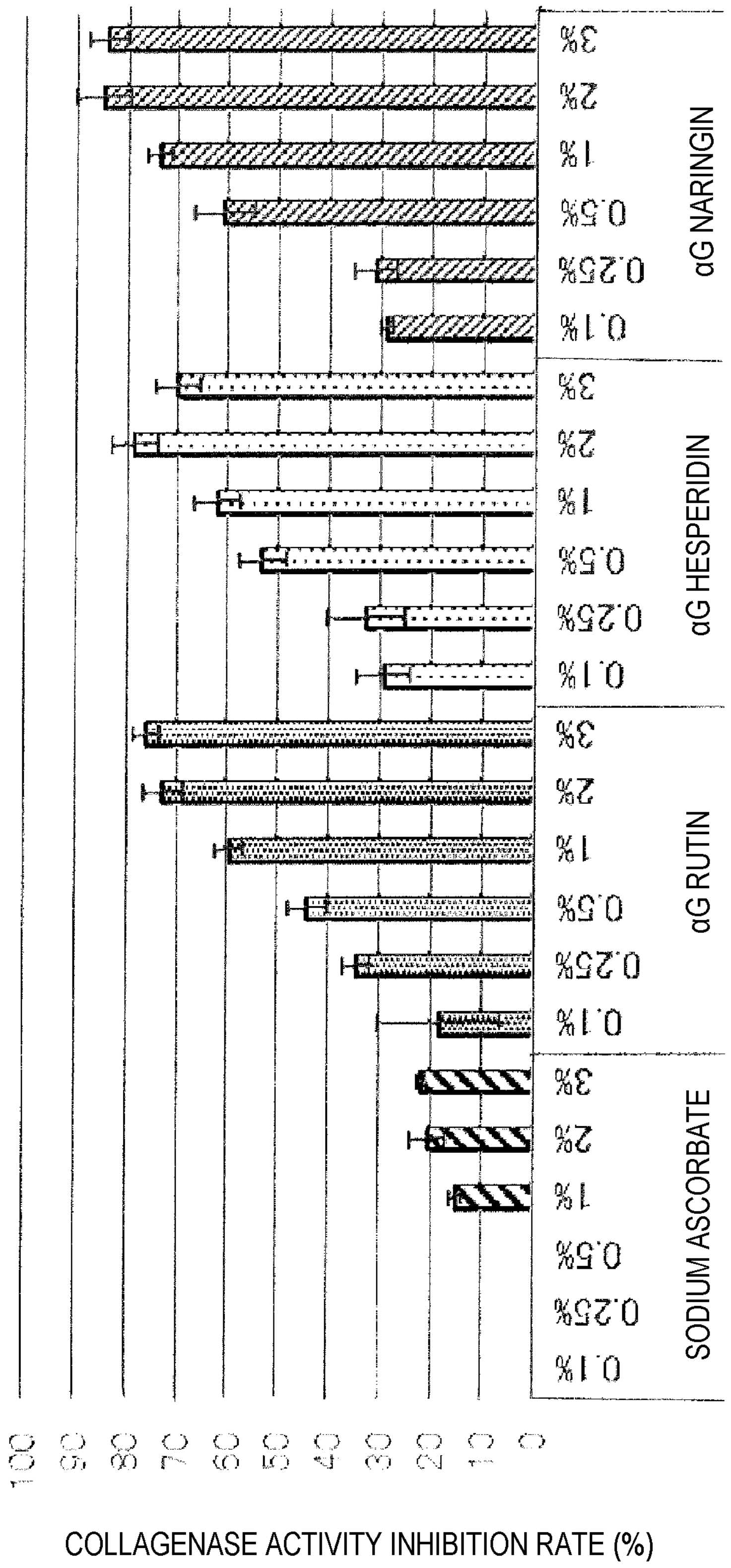
αG NARINGIN
αG HESPERIDIN
αG RUTIN
SODIUM ASCORBATE
0.1%
0.25%
0.5%
1%
2%
3%
100
90
80
70
60
50
40
30
20
10
0
COLLAGENASE ACTIVITY INHIBITION RATE (%)

**INTERNATIONAL SEARCH REPORT**

International application No. PCT/JP2021/011634

A. CLASSIFICATION OF SUBJECT MATTER
A61P 43/00(2006.01)i; A61P 17/00(2006.01)i; A23L 33/105(2016.01)i; A23L 33/125(2016.01)i; A61K 31/7048(2006.01)i
FI: A23L33/125; A23L33/105; A61K31/7048; A61P17/00; A61P43/00 111
According to International Patent Classification (IPC) or to both national classification and IPC

B. FIELDS SEARCHED

Minimum documentation searched (classification system followed by classification symbols)
A23L, A61K, A61P

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY/MEDLINE/EMBASE/BIOSIS (STN), JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X | JP 6-183941 A (LION CORP.) 05 July 1994 (1994-07-05) claim 1, paragraph [0014], examples, tables 1-2 | 1-5 |
| Y | claim 1, paragraph [0014], examples, tables 1-2 | 1-5 |
| X | JP 2009-256341 A (SUNSTAR INC.) 05 November 2009 (2009-11-05) claims, paragraphs [0021]-[0022], examples, table 1 | 1-5 |
| Y | claims, paragraphs [0021]-[0022], examples, table 1 | 1-5 |
| X | JP 2002-234844 A (TOYO SUGAR REFINING CO., LTD.) 23 August 2002 (2002-08-23) claims, paragraphs [0015]-[0031], [0049]-[0054], examples | 1-5 |
| Y | claims, paragraphs [0015]-[0031], [0049]-[0054], examples | 1-5 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 May 2021 (11.05.2021) | 25 May 2021 (25.05.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2021/011634

C (Continuation). DOCUMENTS CONSIDERED TO BE RELEVANT

| Category* | Citation of document, with indication, where appropriate, of the relevant passages | Relevant to claim No. |
|---|---|---|
| Y | LU, Weiqiang et al., "The efficient expression of hU111an fibroblast collagenase in Escherichia coli and the discovery of flavonoid inhibitors", Journal of Enzyme Inhibition and Medicinal Chemistry, 2013, vol. 28, no. 4, pp. 741-746, DOI:10.3109/14756366.2012.681650 abstract, table 1 | 1-5 |
| A | JP 3-58790 A (HAYASHIBARA BIOCHEMICAL LABORATORIES, INC.) 13 March 1991 (1991-03-13) entire text | 1-5 |
| A | JP 2002-199896 A (HAYASHIBARA BIOCHEMICAL LABORATORIES, INC.) 16 July 2002 (2002-07-16) entire text | 1-5 |
| A | JP 10-323196 A (HAYASHIBARA BIOCHEMICAL LABORATORIES, INC.) 08 December 1998 (1998-12-08) entire text | 1-5 |

Form PCT/ISA/210 (continuation of second sheet) (January 2015)

**INTERNATIONAL SEARCH REPORT**
Information on patent family members

International application No.
PCT/JP2021/011634

| Patent Documents referred in the Report | Publication Date | Patent Family | Publication Date |
|---|---|---|---|
| JP 6-183941 A | 05 Jul. 1994 | (Family: none) | |
| JP 2009-256341 A | 05 Nov. 2009 | JP 2015-131830 A | |
| JP 202-234844 A | 23 Aug. 2002 | (Family: none) | |
| JP 3-58790 A | 13 Mar. 1991 | JP 2000-60484 A<br>US 5145781 A1<br>entire text<br>EP 387042 A1<br>DE 69016800 C<br>CA 2011618 A1<br>KR 10-1990-0014421 A<br>KR 10-0165956 B1<br>KR 10-0176956 B1<br>CA 2011618 A1 | |
| JP 2002-199896 A | 16 Jul. 2002 | (Family: none) | |
| JP 10-323196 A | 08 Dec. 1998 | US 6048712 A1<br>entire text<br>WO 1998/042859 A1<br>EP 908524 A1<br>DE 69821428 D<br>TW 521087 B<br>KR 10-1998-0080520 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003183122 A
- JP 2003342123 A
- JP 2006225286 A
- JP 2008074728 A
- JP 2011011991 A
- JP 2016104707 A
- JP H413691 A
- JP 2002199896 A